# EUROPEAN PATENT SPECIFICATION

(11) **EP 4 385 456 B1**
(45) Date of publication and mention of the grant of the patent: **06.05.2026**
(21) Application number: 22856023.1
(22) Date of filing: 12.07.2022
(51) Int. Cl.: A61C 13/00, A61C 9/00, G16H 50/50, A61C 5/70, A61B 34/10

(54) **FACIAL SHAPE CLASSIFICATION METHOD AND DENTURE DESIGN DEVICE THEREFOR**
GESICHTSFORMKLASSIFIZIERUNGSVERFAHREN UND ZAHNERSATZENTWURFSVORRICHTUNG DAFÜR
PROCÉDÉ DE CLASSIFICATION DE FORME DE VISAGE ET DISPOSITIF DE CONCEPTION DENTAIRE ASSOCIÉ

(30) Priority: 13.08.2021 KR 20210107480
(43) Date of publication of application: 19.06.2024
(73) Proprietor: Osstem Implant Co., Ltd., Gangseo-gu Seoul 07789 (KR)
(72) Inventor: KIM, Young Seok, Anyang-si Gyeonggi-do 14069 (KR); CHO, In Ho, Seoul 06317 (KR); CHOI, Kyoo Ok, Seoul 07789 (KR)
(74) Representative: Klunker IP Patentanwälte PartG mbB
(86) International application number: PCT/KR2022/010119
(87) International publication number: WO 2023/018021

(56) References cited:
- EP-B1- 3 047 817
- WO-A1-2018/230873
- KR-A- 20210 056 141
- KR-A- 20210 069 172
- KR-B1- 101 067 942
- US-A1- 2019 117 337
- VASANTHA KUMAR M., AHILA S. C., SUGANYA DEVI S.: "The Science of Anterior Teeth Selection for a Completely Edentulous Patient: A Literature Review", THE JOURNAL OF INDIAN PROSTHODONTIC SOCIETY, SPRINGER INDIA, INDIA, vol. 11, no. 1, 1 March 2011 (2011-03-01), India , pages 7 - 13, XP093034367, ISSN: 0972-4052, DOI: 10.1007/s13191-011-0058-9

## Description

### [Technical Field]

The present invention relates to dental image processing technology, and more specifically, to technology for designing a denture using dental images.

### [Background Art]

Restoration using complete dentures in edentulous patients is a treatment concept entirely different from restorative treatment of natural teeth. A user must set everything, including the positions of artificial teeth, and all teeth move as one unit, with a constant risk of dislodgment. When making such complete dentures, if the optimal artificial teeth are selected and placed, it may become easier to make occlusal adjustments for complete denture patients.

Currently, one method to choose appropriate artificial teeth is for the user to visually inspect the patient's facial photos or model data and make a selection. In this case, the method relies on the user's experience rather than precise data. The conventional method that relies on user experience is cumbersome, as it requires the user to choose the shape of artificial teeth each time, and the results may vary depending on the user's proficiency.

KR 10-1067942 B1 relates to a dental image processing system and method in which patient image data are acquired and processed by a computing device to support dental treatment and prosthesis-related operations. The document describes extracting feature information from image data and performing calculations based on such extracted features in order to derive information relevant for dental applications. In particular, KR 10-1067942 B1 is concerned with computer-assisted processing of patient-related image data and the use of calculated parameters derived from image features to assist dental procedures and decision-making in a digital dental environment.

WO 2018/230873 A1 discloses a tooth shape classifying and dental prosthesis shape guiding method in which images of a patient's teeth are acquired, prosthetic tooth sample images are classified according to shape-related characteristics, and a prosthetic tooth image is selected and applied to generate an expected prosthetically treated tooth image. The document focuses on classification and selection of artificial tooth shapes based on visual characteristics of tooth images and provides a user-guided workflow for selecting and applying prosthetic tooth shapes within a digital dental treatment context.

### [Disclosure]

### [Technical problem]

It is an object of the present invention to provide a facial shape classification method and device which can select the optimal artificial tooth when designing a complete denture, minimize user's manipulation, and increase the patient's aesthetic satisfaction, and a denture design device therefor.

### [Technical Solution]

The object is achieved by the features of independent claim 1 regarding the method and of independent claim 8 regarding the device. Further embodiments are defined in the dependent claims.

### [Advantageous Effects]

According to a facial shape classification method in accordance with the invention and a denture design device therefor, it is possible to minimize user's manipulation by automatically classifying and providing the shape of an artificial tooth in a complete denture production.

In addition, by considering the individual facial shape of a patient to determine and provide the shape of artificial teeth, accuracy and patient aesthetic satisfaction may be enhanced.

Moreover, allowing a user to modify the provided patient's facial shape increases user convenience.

### [Description of Drawings]

FIG. 1 is a diagram illustrating a configuration of a denture design device.
FIG. 2 is a flowchart illustrating a facial shape classification method.
FIG. 3 illustrates facial shapes classified.
FIG. 4 is a table showing facial shape-specific artificial tooth shapes classified.
FIG. 5 is a diagram illustrating an example of user modification to the shape of an artificial tooth provided.
FIG. 6 is a diagram illustrating an example of user modification to the shape of an artificial tooth provided.

### [Mode of the Invention]

The advantages and features of the present invention and the manner of achieving the advantages and features will become apparent with reference to detailed description below together with the accompanying drawings. However, the present invention may be implemented in many different forms and should not be construed as being limited to the description set forth herein. Throughout the entire specification, the same or like reference numerals designate the same or like elements.

In describing examples , a detailed description of related known configurations or functions incorporated herein will be omitted when it is determined that the detailed description thereof may unnecessarily obscure the subject matter of the present invention. The terms which will be described below are terms defined in consideration of the functions in the present invention, and may be different according to users, intentions of the users, or customs. Therefore, the definitions of the terms used herein should follow contexts disclosed herein.

Hereinafter, the present invention will be described in detail with reference to the accompanying drawings.

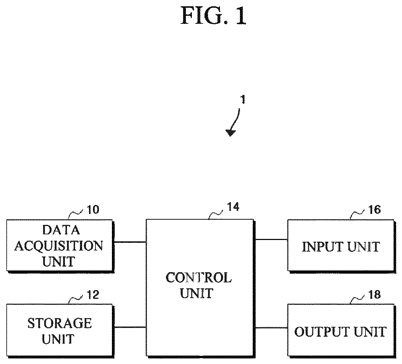

FIG. 1 is a diagram illustrating a configuration of a denture design device.

A denture design device 1 is an electronic device capable of executing image processing programs. Examples of the electronic device include personal computers (PCs), notebook computers, tablet computers, smartphones, mobile phones, and the like. Medical image processing programs include design programs, scanning programs, CAD programs, and the like. These medical image processing programs may be denture design programs for producing complete dentures for edentulous patients, but they may also be applied to other general medical image processing programs. Hereinafter, for the convenience of description, a dental design program will be described as an example, but if image processing is possible, other programs are applicable.

Referring to FIG. 1, the denture design device 1 includes a data acquisition unit 10, a storage unit 12, a control unit 14, an input unit 16, and an output unit 18.

The data acquisition unit 10 acquires dental image data from teeth, including the patient's damaged teeth. The dental image data may include facial shape data such as the patient's facial photos, facial scan data, and the like.

The storage unit 12 stores data, such as information necessary for the operation of the denture design device 1, information generated during its operation, and the like. For example, individual patient scan data may be stored in the storage unit 12 and provided to the control unit 14. The storage unit 12 may store maxillary and mandibular dental image data of individual patients, and provide dental image data of a specific patient to the control unit 14. In addition, artificial tooth shape information for each of the pre-classified facial shapes may be stored in the storage unit 12.

The control unit 14 designs a complete denture under the control of a computer program. The control unit 14 determines the shape of an artificial tooth for complete denture design. Here, the shape includes size.

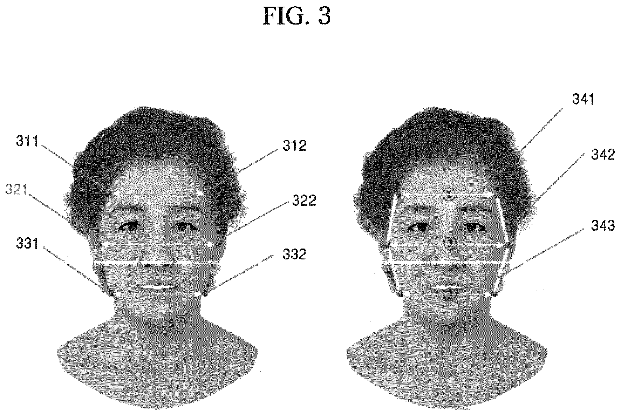

As an example of determining the shape of an artificial tooth by the control unit 14, a plurality of landmarks are extracted from the facial data obtained through the data acquisition unit 10. The plurality of extracted landmarks serve as anatomical reference points in the facial data. For example, these landmarks may include endpoints on both sides of the one-third portion of the forehead area, tragus points on both sides of the face, and gonion points on both sides of the face. The endpoints on both sides of the one-third portion of the forehead area refer to endpoints on both sides of a portion that represents one-third of the entire forehead area (from the glabella to the trichion) when it is equally trisected vertically. The tragi on both sides refer to the cartilaginous prominences situated anteriorly to the entrance of the external auditory canal. The gonia on both sides refer to the angular areas at the rear bottom of the mandible. Examples of the landmarks will be described below with reference to FIG. 3.

Then, the control unit 14 classifies the patient's facial shape using the distances between the extracted landmarks. For example, the control unit 14 calculates a first distance, a second distance, and a third distance between the landmarks and classifies the patient's facial shape based on the comparison of the calculated first, second, and third distances. Here, the first distance is the distance of a first line segment connecting both endpoints of the one-third portion of the forehead area in the facial data. The second distance is the distance of a second line segment connecting both tragus points. The third distance is the distance of a third line segment connecting both gonion points. Examples of the facial shape classification will be described below with reference to FIG. 3.

Subsequently, the control unit 14 classifies the patient's artificial tooth shape according to the classified facial shape and provides it through the output unit 18. For this purpose, artificial tooth shapes matching the facial shapes are pre-stored in the storage unit 12, and the control unit 14 selects a specific artificial tooth shape that matches the classified facial shape from the multiple artificial tooth shapes stored in the storage unit 12.

The control unit 14 may classify the artificial tooth shape as at least one of square, tapering, or ovoid, or a combination of at least two thereof according to the facial shape. For example, the artificial tooth shape may be classified as square, tapering, ovoid, square-tapering, tapering-ovoid, ovoid-square, or square-tapering-ovoid.

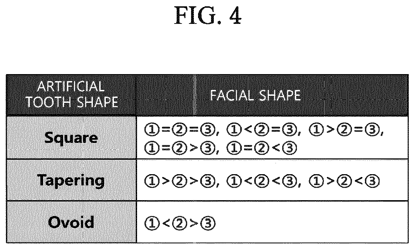

For example, if the facial shape is such that first distance=the second distance=the third distance, or first distance<second distance=third distance, or first distance>second distance=third distance, or first distance=second distance<third distance, the control unit 14 may classify the artificial tooth shape as square. If the facial shape is such that first distance>second distance>third distance, or first distance<second distance<third distance, or first distance>second distance<third distance, the control unit 14 may classify the artificial tooth shape as tapering. If the facial form is such that first distance<second distance>third distance, the control unit 14 may classify the artificial tooth shape as ovoid. Examples of the facial shape classification will be described below with reference to FIG. 4. When the first distance, the second distance, or the third distance is equal to each other, it may imply that if the difference between distances falls within a predetermined margin of error, they are considered equal. For example, if the control unit 14 detects a difference of less than 5 mm between two distances, it may be considered that these distances are equal. In another example, the control unit 14 may determine whether the distances are equal to each other based on a comparison of distances after truncating decimal places in centimeters when comparing distances.

If there are multiple classified artificial tooth shapes, the control unit 14 may provide multiple artificial tooth shapes through the output unit 18. When the user selects a specific artificial tooth shape through user input via the input unit 16, the control unit 14 may provide the selected artificial tooth shape through the output unit 18.

When the input unit 16 receives a user selection signal for modification to the artificial tooth shape, the control unit 14 may modify the artificial tooth shape, and the output unit 18 may display the modified artificial tooth shape.

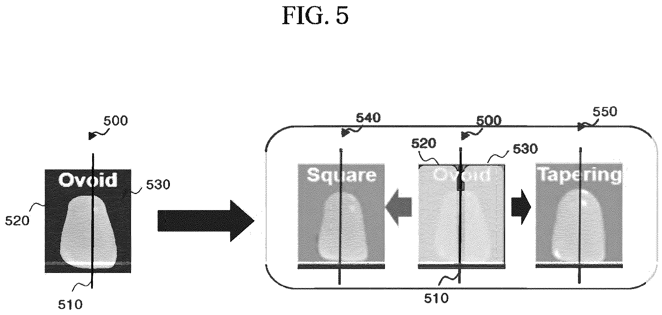

For example, when providing a first artificial tooth shape, the control unit 14 divides the first artificial tooth shape vertically into right and left halves and displays the divided first artificial tooth shape and the dividing line through the output unit 18. In this case, if the user selects either the left or right half through a manipulation signal based on a user action via the input unit 16, the control unit 14 provides the modified artificial tooth shape in which, when the right half is selected, the right half is modified to a second artificial tooth shape, or, when the left half is selected, the left half is modified to a third artificial tooth shape, through the output unit 18. An example of this will be described below with reference to FIG. 5.

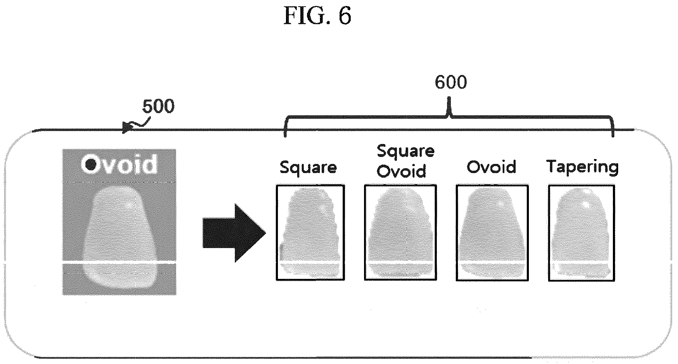

In another example, when the artificial tooth shape is selected by a user action, the control unit 14 displays an artificial tooth replacement list on a screen through the output unit 18. In this case, if the input unit 16 receives a specific artificial tooth shape that the user selects from the artificial tooth replacement list, the control unit 14 modifies the artificial tooth shape to the selected one and provides the modified artificial tooth shape through the output unit 18. An example of this will be described below with reference to FIG. 6.

The input unit 16 receives a user manipulation signal. For example, the input unit 16 receives the manipulation signal based on a user action (e.g., a mouse click) for modifying the artificial tooth shape that is performed for the artificial tooth shape displayed as a virtual graphical object on the screen through the output unit 18.

The output unit 18 displays data on the screen and displays a simulation process conducted through the control unit 14. In this case, the artificial tooth shape designed by the control unit 14 may be displayed on the screen and output through a 3D printer.

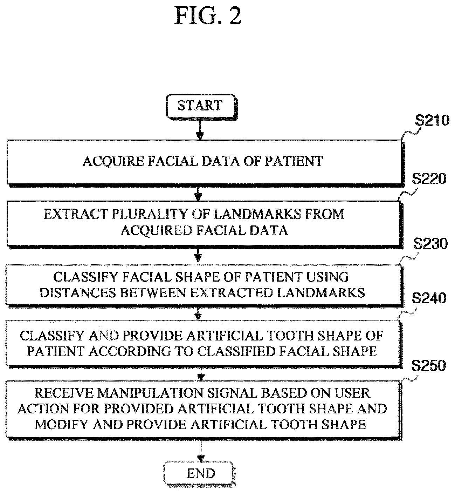

FIG. 2 is a flowchart illustrating a facial shape classification method.

Referring to FIGS. 1 and 2, the denture design device 1 acquires the patient's facial data (S210). Subsequently, the denture design device 1 extracts a plurality of landmarks from the acquired facial data (S220). The plurality of landmarks may be at least one of endpoints on both sides of the one-third portion of the forehead area, tragus points on both sides of the face, or gonion points on both sides of the face in the facial data.

Thereafter, the denture design device 1 classifies the facial shape of a patient using the distances between the extracted landmarks (S230). In the classifying of the facial shape of a patient (S230), the denture design device 1 may calculate the first distance of the first line segment connecting both endpoints of one-third of the forehead area, the second distance of the second line segment connecting the tragus points on both sides, and the third distance of the third line segment connecting the gonion points on both sides. Then, the denture design device may 1 classify the patient's facial shape based on the comparison of the calculated first, second, and third distances.

Subsequently, the denture design device 1 classifies and provides the patient's artificial tooth shape according to the classified facial shape (S240).

In the classifying of the patient's artificial tooth shape (S240), the denture design device 1 may classify the artificial tooth shape as at least one of square, tapering, or ovoid, or a combination of at least two thereof according to the facial shape. For example, if the facial shape is such that first distance=second distance=third distance, or first distance<second distance=third distance, or first distance>second distance=third distance, or first distance=second distance<third distance, the denture design device 1 may classify the artificial tooth shape as square. If the facial shape is such that first distance>second distance>third distance, or first distance<second distance<third distance, or first distance>second distance<third distance, the denture design device 1 may classify the artificial tooth shape as tapering. If the facial form is such that first distance<second distance>third distance, the denture design device 1 may classify the artificial tooth shape as ovoid.

In the classifying of the artificial tooth shape (S240), if there are multiple classified artificial tooth shapes, the denture design device 1 may provide the multiple artificial tooth shapes, receives a specific artificial tooth shape selected from among the multiple provided artificial tooth shapes through a user manipulation, and provide the selected artificial tooth shape.

Furthermore, the denture design device 1 may receive a manipulation signal based on the user action for the provided artificial tooth shape and modify and provide the artificial tooth shape (S250).

In the modifying of the artificial tooth shape (S250), when providing a first artificial tooth shape, the denture design device 1 may divide the first artificial tooth shape vertically into right and left halves and display the dividing line. In this case, if either the left or right half is selected by a manipulation signal based on the user action, the dental design device 1 may provide the modified artificial tooth shape in which, when the right half is selected, the right half is modified to a second artificial tooth shape, or, when the left half is selected, the left half is modified to a third artificial tooth shape.

In the modifying of the artificial tooth shape (S250), the denture design device 1 may display an artificial tooth replacement list when the user selects a specific artificial tooth shape. In this case, if the user selects a specific artificial tooth shape from the artificial tooth replacement list, the artificial tooth shape may be modified to the selected artificial tooth shape and provided.

FIG. 3 illustrates facial shapes classified.

Referring to FIGS. 1 and 3, the denture design device 1 classifies the patient's facial shape using the plurality of landmarks extracted from the facial data.

As shown in FIG. 3, the denture design device 1 may extract six landmarks from the patient's facial data. These six landmarks may include, for example, endpoints 311 and 312 on both sides of the one-third portion of the forehead area, tragi 321 and 322 on both sides of the face, and gonia 331 and 332 on both sides of the face. The endpoints 311 and 312 on both sides of the one-third portion of the forehead area refer to endpoints on both sides of a portion that represents one-third of the entire forehead area when it is equally trisected vertically. The tragi 321 and 322 on both sides refer to the cartilaginous prominences situated anteriorly to the entrance of the external auditory canal. The gonia 331 and 332 on both sides refer to the angular areas at the rear bottom of the mandible.

The denture design device 1 calculates a distance of a line segment created by connecting two points of the landmarks of the facial shape. For example, the denture design device 1 calculates the first distance① 341 of the first line segment connecting the endpoints 311 and 312 on both sides of the one-third portion of the forehead area, the second distance② 342 of the second line segment connecting the tragi 321 and 322 on both sides, and the third distance③ 343 of the third line segment connecting the gonia 331 and 332 on both sides.

Subsequently, the denture design device 1 may classify the patient's facial shape based on the comparison of the calculated first distance① 341, second distance② 342, and third distance③ 343. For example, the facial shape based on the comparison of the first distance① 341, the second distance② 342, and the third distance③ 343 may be classified into nine types, such as ①=②=③, ①=②>③, ①=②<③, ①>②=③, ①>②>③, ①>②<③, ①<②>③, ①<②<③, and ①<②=③.

FIG. 4 is a table showing facial shape-specific artificial tooth shapes classified.

Referring to FIGS. 1 and 4, the denture design device 1 classifies the artificial tooth shape according to the patient's facial shape and provides it to the user. For this purpose, the denture design device 1 pre-classifies and stores artificial tooth shapes matching the facial shapes in the storage unit 12. When the facial shape is determined, the denture design device 1 searches for the artificial tooth shape in the storage unit 12 that matches the classified facial shape and provides it. For example, as shown in FIG. 4, the denture design device 1 classifies nine facial shapes into three artificial tooth shapes. The three artificial tooth shapes include square, tapering, and ovoid. For example, if the facial shape is ①=②=③, or ①<②=③, or ①>②=③, or ①=②>③, or ①=②<③, the denture design device 1 may classify the artificial tooth shape as square. If the facial shape is ①>②>③, or ①<②<③, or ①>②<③, the denture design device 1 may classify the artificial tooth shape as tapering. If the facial shape is ①<②>③, the denture design device 1 may classify the artificial tooth shape as ovoid.

In another example, in addition to the aforementioned three shapes, the artificial tooth shapes may be classified into other types, such as four, seven categories, or the like. For example, artificial tooth shapes may be classified into four categories, including square, tapering, ovoid, and square-ovoid. Similar to the combination of square and ovoid, at least two artificial tooth shapes may be combined, resulting in artificial tooth shape classifications such as square-ovoid, square-tapering, square-tapering-ovoid, and the like.

The criteria for classifying artificial tooth shapes based on facial shapes are pre-set and stored in the storage unit 12, and they may be modified by the user.

FIG. 5 is a diagram illustrating an example of user modification to the shape of an artificial tooth provided.

Referring to FIGS. 1 and 5, the user may modify an artificial tooth shape provided by the denture design device 1. For example, the denture design device 1 vertically divides the provided artificial tooth shape 500 into a left half 520 and a right half 530, and displays a dividing line 510. In this case, when the user selects either the left half or the right half through a manipulation signal based on a user action (e.g., a mouse click), the right half may be used as a "next" button, while the left half may be used as a "previous" button. For example, in the case where the artificial tooth shapes include square, ovoid, and tapering shapes and the denture design device 1 provides an ovoid shape 500, if the user selects the left half 520 of the provided ovoid shape 500 based on the dividing line 510, the denture design device 1 modifies the artificial tooth shape from the ovoid shape 500 to a square shape 540 and provides the modified shape. Conversely, if the user selects the right half 530 of the provided ovoid shape 500 based on the dividing line 510, the denture design device 1 modifies the artificial tooth shape from the ovoid shape 500 to a tapering shape 550 and provides the modified shape. When the user makes modifications, the denture design device 1 may display both the original artificial tooth shape 500 and the modified artificial tooth shape 540 or 550 in a visually distinguished manner. In addition, when the left half 520 or the right half 530 based on the dividing line of the artificial tooth shape 500 is selected, the denture design device 1 may display the selected half in a visually distinguished manner.

In the example shown in FIG. 5, when there are three different artificial tooth shapes, the artificial tooth shape may be modified with a single selection action (e.g., a click) of the user.

As shown in FIG. 5, the artificial tooth shape may be modified through the user action for selecting either the left or right side of the provided artificial tooth. However, an additional graphical user interface may be provided around the provided artificial tooth for user selection and the artificial tooth shape may be modified based on the user's selection through the provided graphical user interface.

FIG. 6 is a diagram illustrating an example of user modification to the shape of an artificial tooth provided.

Referring to FIGS. 1 and 6, even when there are four or more classification criteria for artificial tooth shapes, the user may still make modifications. For example, when there are at least four classification criteria for artificial tooth shapes, if a user selects a specific artificial tooth shape, for example, an ovoid shape 500, through a user action (e.g., a click) the denture design device 1 displays an artificial tooth replacement list 600 on a screen. In this case, when the user selects a specific artificial tooth shape from the artificial tooth replacement list 600 through a user action (e.g., a click), the denture design device 1 may modify the artificial tooth shape to the artificial tooth shape selected by the user and provide the modified artificial tooth shape.

In the example shown in FIG. 6, when there are four or more artificial tooth shapes, the artificial tooth shape may be modified with two selection actions (e.g., clicks) of the user.

## Claims

1. A facial shape classification method, which is performed using a denture design device (1), comprising:
in the denture design device (1), acquiring facial data of a patient;
extracting a plurality of landmarks (311, 312, 321, 322, 331, 332) from the acquired facial data;
classifying a facial shape of the patient using distances (341, 342, 343) between the plurality of extracted landmarks (311, 312, 321, 322, 331, 332); and
classifying and providing an artificial tooth shape of the patient according to the classified facial shape;
**characterized in that**
the extracting of the plurality of landmarks (311, 312, 321, 322, 331, 332) comprises extracting, as the plurality of landmarks,
(i) endpoints (311, 312) on both sides of the one-third portion of a forehead area, and
(ii) tragus points (321, 322) on both sides of the face, and
(iii) gonion points (331, 332) on both sides of the face ; and
the classifying of the facial shape of a patient comprises:
calculating a first distance (341) of a first line segment connecting endpoints (311, 312) on both sides of the one-third portion of the forehead area in the facial data, a second distance (342) of a second line segment connecting the tragus points (321, 322) on both sides, and a third distance (343) of a third line segment connecting the gonion points (331, 332) on both sides; and
classifying the facial shape of the patient based on a comparison of the calculated first, second, and third distances (341, 342, 343).

2. The facial shape classification method of claim 1, wherein in the classifying and providing of the artificial tooth shape of a patient, the artificial tooth shape is classified as at least one of square, tapering, or ovoid, or a combination of at least two thereof according to the facial shape.

3. The facial shape classification method of claim 1, wherein in the classifying and providing of the artificial tooth shape of a patient,
when the facial shape is such that
- first distance (341) equals the second distance (342) and the second distance (342) equals the third distance (343) , or
- the first distance (341) is smaller than the second distance (342) and the second distance (342) equals the third distance (343), or
- the first distance (341) is greater than the second distance (342) and the second distance (342) equals the third distance (343), or
- the first distance (341) equals the second distance (342) and the second distance (342) is smaller than the third distance (343),
the artificial tooth shape is classified as square;
when the facial shape is such that
- the first distance (341) is greater than the second distance (342) and the second distance (342) is greater than the third distance (343), or
- the first distance (341) is smaller than the second distance (342) and the second distance (342) is smaller than the third distance (343), or
the first distance (341) is greater than the second distance (342) and the second distance (342) is smaller than the third distance (343),
the artificial tooth shape is classified as tapering; and
when the facial form is such that the first distance (341) is smaller than the second distance (342) and the second distance (342) is greater than the third distance (343), the artificial tooth shape is classified as ovoid,
wherein the first distance (341) is the distance of the first line segment connecting the endpoints (311, 312) on both sides of the one-third portion of the forehead area in the facial data, the second distance (342) is the distance of the second line segment connecting the tragus points (321, 322) on both sides, and the third distance (343) is the distance of the third line segment connecting the gonion points (331, 332) on both sides.

4. The facial shape classification method of claim 1, further comprising, when there are multiple classified artificial tooth shapes, providing the multiple artificial tooth shapes, receiving a specific artificial tooth shape selected from among the multiple provided artificial tooth shapes through a user action, and providing the selected artificial tooth shape.

5. The facial shape classification method of claim 1, further comprising receiving a manipulation signal based on a user action for the provided artificial tooth shape, and modifying and providing the artificial tooth shape.

6. The facial shape classification method of claim 5, wherein the modifying and providing of the artificial tooth shape comprises,
when providing a first artificial tooth shape (500), vertically dividing the first artificial tooth shape (500) into right and left halves (530, 520) while displaying a dividing line (510), and
in response to selecting either the left half (520) or the right half (530) through a manipulation signal based on a user action, providing a modified artificial tooth shape (540, 550) in which, when the right half (530) is selected, the right half (530) is modified to a second artificial tooth shape (540), or, when the left half (520) is selected, the left half (520) is modified to a third artificial tooth shape (550).

7. The facial shape classification method of claim 5, wherein the modifying and providing of the artificial tooth shape comprises,
in response to selecting a specific artificial tooth shape (500) through a user manipulation, displaying an artificial tooth replacement list (600) on a screen and
in response to selecting a specific artificial tooth shape from the artificial tooth replacement list (600) through a user manipulation, modifying the artificial tooth shape to the selected artificial tooth shape and providing the modified artificial tooth shape.

8. A denture design device (1) comprising:
a data acquisition unit (10) configured to acquire facial data of a patient;
a control unit (14) configured to extract a plurality of landmarks (311, 312, 321, 322, 331, 332) from the acquired facial data, to classify a facial shape of the patient using distances (341, 342, 343) between the plurality of extracted landmarks (311, 312, 321, 322, 331, 332), and to classify an artificial tooth shape of the patient according to the classified facial shape; and
an output unit (18) configured to provide the classified artificial tooth shape,
**characterized in that**
the control unit (14) is configured to extract, as the plurality of landmarks,
(i) endpoints (311, 312) on both sides of a one-third portion of a forehead area in the facial data,
(ii) tragus points (321, 322) on both sides of the face, and
(iii) gonion points (331, 332) on both sides of the face, and
the control unit (14) is configured to classify the facial shape of the patient by:
calculating a first distance (341) of a first line segment connecting the endpoints (311, 312) on both sides of the one-third portion of the forehead area, a second distance (342) of a second line segment connecting the tragus points (321, 322) on both sides, and a third distance (343) of a third line segment connecting the gonion points (331, 332) on both sides; and
classifying the facial shape of the patient based on a comparison of the calculated first, second, and third distances (341, 432, 433).

## Patentansprüche

1. Gesichtsformklassifizierungsverfahren, das unter Verwendung einer Prothesengestaltungsvorrichtung (1) durchgeführt wird, aufweisend:
in der Prothesengestaltungsvorrichtung (1), Erfassen von Gesichtsdaten eines Patienten;
Extrahieren einer Mehrzahl von Landmarken (311, 312, 321, 322, 331, 332) aus den erfassten Gesichtsdaten;
Klassifizieren einer Gesichtsform des Patienten unter Verwendung von Abständen (341, 342, 343) zwischen der Mehrzahl von extrahierten Landmarken (311, 312, 321, 322, 331, 332); und
Klassifizieren und Bereitstellen einer Kunstzahnform des Patienten gemäß der klassifizierten Gesichtsform;
**dadurch gekennzeichnet, dass**
das Extrahieren der Mehrzahl von Landmarken (311, 312, 321, 322, 331, 332) aufweist ein Extrahieren, als die Mehrzahl von Landmarken, von
(i) Endpunkten (311, 312) auf beiden Seiten des Drittelabschnitts eines Stirnbereichs, und
(ii) Traguspunkten (321, 322) auf beiden Seiten des Gesichts, und
(iii) Gonionpunkten (331, 332) auf beiden Seiten des Gesichts; und
das Klassifizieren der Gesichtsform eines Patienten aufweist:
Berechnen eines ersten Abstands (341) eines ersten Liniensegments, das die Endpunkte (311, 312) auf beiden Seiten des Drittelabschnitts des Stirnbereichs in den Gesichtsdaten verbindet, eines zweiten Abstands (342) eines zweiten Liniensegments, das die Traguspunkte (321, 322) auf beiden Seiten verbindet, und eines dritten Abstands (343) eines dritten Liniensegments, das die Gonionpunkte (331, 332) auf beiden Seiten verbindet; und
Klassifizieren der Gesichtsform des Patienten basierend auf einem Vergleich der berechneten ersten, zweiten und dritten Abstände (341, 342, 343).

2. Gesichtsformklassifizierungsverfahren nach Anspruch 1, wobei beim Klassifizieren und Bereitstellen der Kunstzahnform eines Patienten die Kunstzahnform als mindestens eine von quadratisch, spitz zulaufend oder ovoid, oder als eine Kombination von mindestens zwei davon gemäß der Gesichtsform klassifiziert wird.

3. Gesichtsformklassifizierungsverfahren nach Anspruch 1, wobei beim Klassifizieren und Bereitstellen der Kunstzahnform eines Patienten,
wenn die Gesichtsform derart ist, dass
der erste Abstand (341) gleich dem zweiten Abstand (342) ist und der zweite Abstand (342) gleich dem dritten Abstand (343) ist, oder
der erste Abstand (341) kleiner als der zweite Abstand (342) ist und der zweite Abstand (342) gleich dem dritten Abstand (343) ist, oder
der erste Abstand (341) größer als der zweite Abstand (342) ist und der zweite Abstand (342) gleich dem dritten Abstand (343) ist, oder
der erste Abstand (341) gleich dem zweiten Abstand (342) ist und der zweite Abstand (342) kleiner als der dritte Abstand (343) ist,
die Kunstzahnform als quadratisch klassifiziert wird;
wenn die Gesichtsform derart ist, dass
der erste Abstand (341) größer als der zweite Abstand (342) ist und der zweite Abstand (342) größer als der dritte Abstand (343) ist, oder
der erste Abstand (341) kleiner als der zweite Abstand (342) ist und der zweite Abstand (342) kleiner als der dritte Abstand (343) ist, oder
der erste Abstand (341) größer als der zweite Abstand (342) ist und der zweite Abstand (342) kleiner als der dritte Abstand (343) ist,
die Kunstzahnform als spitz zulaufend klassifiziert wird; und
wenn die Gesichtsform derart ist, dass der erste Abstand (341) kleiner als der zweite Abstand (342) ist und der zweite Abstand (342) größer als der dritte Abstand (343) ist, die Kunstzahnform als ovoid klassifiziert wird,
wobei der erste Abstand (341) der Abstand des ersten Liniensegments ist, das die Endpunkte (311, 312) auf beiden Seiten des Drittelabschnitts des Stirnbereichs in den Gesichtsdaten verbindet, der zweite Abstand (342) der Abstand des zweiten Liniensegments ist, das die Traguspunkte (321, 322) auf beiden Seiten verbindet, und der dritte Abstand (343) der Abstand des dritten Liniensegments ist, das die Gonionpunkte (331, 332) auf beiden Seiten verbindet.

4. Gesichtsformklassifizierungsverfahren nach Anspruch 1, ferner aufweisend, wenn es mehrere klassifizierte Kunstzahnformen gibt, Bereitstellen der mehreren Kunstzahnformen, Empfangen einer bestimmten Kunstzahnform, die aus den mehreren bereitgestellten Kunstzahnformen durch eine Benutzeraktion ausgewählt wird, und Bereitstellen der ausgewählten Kunstzahnform.

5. Gesichtsformklassifizierungsverfahren nach Anspruch 1, ferner aufweisend Empfangen eines Manipulationssignals basierend auf einer Benutzeraktion für die bereitgestellte Kunstzahnform und Modifizieren und Bereitstellen der Kunstzahnform.

6. Gesichtsformklassifizierungsverfahren nach Anspruch 5, wobei das Modifizieren und Bereitstellen der Kunstzahnform aufweist:
beim Bereitstellen einer ersten Kunstzahnform (500), vertikales Unterteilen der ersten Kunstzahnform (500) in eine rechte und eine linke Hälfte (530, 520) unter Anzeige einer Trennlinie (510), und
als Reaktion auf ein Auswählen entweder der linken Hälfte (520) oder der rechten Hälfte (530) durch ein Manipulationssignal basierend auf einer Benutzeraktion, Bereitstellen einer modifizierten Kunstzahnform (540, 550), bei der, wenn die rechte Hälfte (530) ausgewählt wird, die rechte Hälfte (530) zu einer zweiten Kunstzahnform (540) modifiziert wird, oder, wenn die linke Hälfte (520) ausgewählt wird, die linke Hälfte (520) zu einer dritten Kunstzahnform (550) modifiziert wird.

7. Gesichtsformklassifizierungsverfahren nach Anspruch 5, wobei das Modifizieren und Bereitstellen der Kunstzahnform aufweist:
als Reaktion auf ein Auswählen einer bestimmten Kunstzahnform (500) durch eine Benutzermanipulation, Anzeigen einer Kunstzahnersetzungsliste (600) auf einem Bildschirm, und
als Reaktion auf ein Auswählen einer bestimmten Kunstzahnform aus der Kunstzahnersetzungsliste (600) durch eine Benutzermanipulation, Modifizieren der Kunstzahnform zu der ausgewählten Kunstzahnform und Bereitstellen der modifizierten Kunstzahnform.

8. Prothesengestaltungsvorrichtung (1), aufweisend:
eine Datenerfassungseinheit (10), die konfiguriert ist, Gesichtsdaten eines Patienten zu erfassen;
eine Steuereinheit (14), die konfiguriert ist, eine Mehrzahl von Landmarken (311, 312, 321, 322, 331, 332) aus den erfassten Gesichtsdaten zu extrahieren, eine Gesichtsform des Patienten unter Verwendung von Abständen (341, 342, 343) zwischen der Mehrzahl von extrahierten Landmarken (311, 312, 321, 322, 331, 332) zu klassifizieren und eine Kunstzahnform des Patienten gemäß der klassifizierten Gesichtsform zu klassifizieren; und
eine Ausgabeeinheit (18), die konfiguriert ist, die klassifizierte Kunstzahnform bereitzustellen,
**dadurch gekennzeichnet, dass**
die Steuereinheit (14) konfiguriert ist, als die Mehrzahl von Landmarken
(i) Endpunkte (311, 312) auf beiden Seiten eines Drittelabschnitts eines Stirnbereichs in den Gesichtsdaten,
(ii) Traguspunkte (321, 322) auf beiden Seiten des Gesichts und
(iii) Gonionpunkte (331, 332) auf beiden Seiten des Gesichts zu extrahieren, und
die Steuereinheit (14) konfiguriert ist, die Gesichtsform des Patienten zu klassifizieren durch:
Berechnen eines ersten Abstands (341) eines ersten Liniensegments, das die Endpunkte (311, 312) auf beiden Seiten des Drittelabschnitts des Stirnbereichs verbindet, eines zweiten Abstands (342) eines zweiten Liniensegments, das die Traguspunkte (321, 322) auf beiden Seiten verbindet, und eines dritten Abstands (343) eines dritten Liniensegments, das die Gonionpunkte (331, 332) auf beiden Seiten verbindet; und
Klassifizieren der Gesichtsform des Patienten basierend auf einem Vergleich der berechneten ersten, zweiten und dritten Abstände (341, 432, 433).

## Revendications

1. Procédé de classification de forme de visage, lequel est réalisé en utilisant un dispositif de conception de prothèse dentaire (1), comprenant :
dans le dispositif de conception de prothèse dentaire (1), l'acquisition de données de visage d'un patient ;
l'extraction d'une pluralité de points de repère (311, 312, 321, 322, 331, 332) à partir des données de visage acquises ;
la classification d'une forme de visage du patient en utilisant des distances (341, 342, 343) entre la pluralité de points de repère extraits (311, 312, 321, 322, 331, 332) ; et
la classification et la fourniture d'une forme de dent artificielle du patient en fonction de la forme de visage classifiée ;
**caractérisé en ce que** :
l'extraction de la pluralité de points de repère (311, 312, 321, 322, 331, 332) comprend l'extraction, en tant que pluralité de points de repère,
(i) de points d'extrémité (311, 312) sur les deux côtés du tiers d'une région frontale, et
(ii) de points de tragus (321, 322) sur les deux côtés du visage, et
(iii) de points de gonion (331, 332) sur les deux côtés du visage ; et
la classification de la forme de visage d'un patient comprend :
le calcul d'une première distance (341) d'un premier segment de droite qui relie des points d'extrémité (311, 312) sur les deux côtés du tiers de la région frontale dans les données de visage, d'une deuxième distance (342) d'un deuxième segment de droite qui relie les points de tragus (321, 322) sur les deux côtés et d'une troisième distance (343) d'un troisième segment de droite qui relie les points de gonion (331, 332) sur les deux côtés ; et
la classification de la forme de visage du patient sur la base d'une comparaison des première, deuxième et troisième distances calculées (341, 342, 343).

2. Procédé de classification de forme de visage selon la revendication 1, dans lequel, lors de la classification et de la fourniture de la forme de dent artificielle d'un patient, la forme de dent artificielle est classifiée en tant qu'au moins une forme parmi une forme carrée, une forme conique et une forme ovoïde ou en tant que combinaison d'au moins deux de ces formes en fonction de la forme de visage.

3. Procédé de classification de forme de visage selon la revendication 1, dans lequel, lors de la classification et de la fourniture de la forme de dent artificielle d'un patient,
lorsque la forme de visage est telle que :
- la première distance (341) est égale à la deuxième distance (342) et la deuxième distance (342) est égale à la troisième distance (343), ou
- la première distance (341) est plus petite que la deuxième distance (342) et la deuxième distance (342) est égale à la troisième distance (343), ou
- la première distance (341) est plus grande que la deuxième distance (342) et la deuxième distance (342) est égale à la troisième distance (343), ou
- la première distance (341) est égale à la deuxième distance (342) et la deuxième distance (342) est plus petite que la troisième distance (343),
la forme de dent artificielle est classifiée en tant que forme carrée ;
lorsque la forme de visage est telle que :
- la première distance (341) est plus grande que la deuxième distance (342) et la deuxième distance (342) est plus grande que la troisième distance (343), ou
- la première distance (341) est plus petite que la deuxième distance (342) et la deuxième distance (342) est plus petite que la troisième distance (343), ou
- la première distance (341) est plus grande que la deuxième distance (342) et la deuxième distance (342) est plus petite que la troisième distance (343),
la forme de dent artificielle est classifiée en tant que forme conique ; et
lorsque la forme de visage est telle que :
- la première distance (341) est plus petite que la deuxième distance (342) et la deuxième distance (342) est plus grande que la troisième distance (343),
la forme de dent artificielle est classifiée en tant que forme ovoïde,
dans lequel la première distance (341) est la distance du premier segment de droite qui relie les points d'extrémité (311, 312) sur les deux côtés du tiers de la région frontale dans les données de visage, la deuxième distance (342) est la distance du deuxième segment de droite qui relie les points de tragus (321, 322) sur les deux côtés et la troisième distance (343) est la distance du troisième segment de droite qui relie les points de gonion (331, 332) sur les deux côtés.

4. Procédé de classification de forme de visage selon la revendication 1, comprenant en outre, lorsqu'il y a de multiples formes de dent artificielle classifiées, la fourniture des multiples formes de dent artificielle, la réception d'une forme de dent artificielle spécifique qui est sélectionnée parmi les multiples formes de dent artificielle fournies par l'intermédiaire d'une action d'utilisateur et la fourniture de la forme de dent artificielle sélectionnée.

5. Procédé de classification de forme de visage selon la revendication 1, comprenant en outre la réception d'un signal de manipulation qui est basé sur une action d'utilisateur pour la forme de dent artificielle fournie et la modification et la fourniture de la forme de dent artificielle.

6. Procédé de classification de forme de visage selon la revendication 5, dans lequel la modification et la fourniture de la forme de dent artificielle comprend :
lors de la fourniture d'une première forme de dent artificielle (500), la division verticalement de la première forme de dent artificielle (500) selon des moitiés droite et gauche (530, 520) tout en affichant une ligne de division (510) ; et
en réponse à la sélection de soit la moitié gauche (520), soit la moitié droite (530) par l'intermédiaire d'un signal de manipulation qui est basé sur une action d'utilisateur, la fourniture d'une forme de dent artificielle modifiée (540, 550) de telle sorte que, lorsque la moitié droite (530) est sélectionnée, la moitié droite (530) soit modifiée selon une deuxième forme de dent artificielle (540) ou que, lorsque la moitié gauche (520) est sélectionnée, la moitié gauche (520) soit modifiée selon une troisième forme de dent artificielle (550).

7. Procédé de classification de forme de visage selon la revendication 5, dans lequel la modification et la fourniture de la forme de dent artificielle comprend :
en réponse à la sélection d'une forme de dent artificielle spécifique (500) par l'intermédiaire d'une manipulation d'utilisateur, l'affichage d'une liste de remplacement de dent artificielle (600) sur un écran ; et
en réponse à la sélection d'une forme de dent artificielle spécifique au sein de la liste de remplacement de dent artificielle (600) par l'intermédiaire d'une manipulation d'utilisateur, la modification de la forme de dent artificielle selon la forme de dent artificielle sélectionnée et la fourniture de la forme de dent artificielle modifiée.

8. Dispositif de conception de prothèse dentaire (1) comprenant :
une unité d'acquisition de données (10) configurée pour acquérir des données de visage d'un patient ;
une unité de commande (14) configurée pour extraire une pluralité de points de repère (311, 312, 321, 322, 331, 332) à partir des données de visage acquises, afin de classifier une forme de visage du patient en utilisant des distances (341, 342, 343) entre la pluralité de points de repère extraits (311, 312, 321, 322, 331, 332) et afin de classifier une forme de dent artificielle du patient en fonction de la forme de visage classifiée ; et
une unité d'émission en sortie (18) configurée pour fournir la forme de dent artificielle classifiée,
**caractérisé en ce que** :
l'unité de commande (14) est configurée pour extraire, en tant que pluralité de points de repère,
(i) des points d'extrémité (311, 312) sur les deux côtés du tiers d'une région frontale dans les données de visage, et
(ii) des points de tragus (321, 322) sur les deux côtés du visage, et
(iii) des points de gonion (331, 332) sur les deux côtés du visage ; et
l'unité de commande (14) est configurée pour classifier la forme de visage du patient en :
calculant une première distance (341) d'un premier segment de droite qui relie les points d'extrémité (311, 312) sur les deux côtés du tiers de la région frontale, une deuxième distance (342) d'un deuxième segment de droite qui relie les points de tragus (321, 322) sur les deux côtés et une troisième distance (343) d'un troisième segment de droite qui relie les points de gonion (331, 332) sur les deux côtés ; et en
classifiant la forme de visage du patient sur la base d'une comparaison des première, deuxième et troisième distances calculées (341, 342, 343).
